(19) Europäisches Patentamt
European Patent Office
Office européen des brevets

(11) Publication number: **0 265 381 B1**

(12) # EUROPEAN PATENT SPECIFICATION

(45) Date of publication of patent specification: **29.05.91**

(51) Int. Cl.⁵: **A01N 59/00**, A61L 2/18, //G02C13/00

(21) Application number: **87810583.2**

(22) Date of filing: **09.10.87**

The file contains technical information submitted after the application was filed and not included in this specification

(54) **A method of disinfecting soft contact lenses with a stabilized hydrogen peroxide solution.**

(30) Priority: **14.10.86 US 917947**

(43) Date of publication of application:
**27.04.88 Bulletin 88/17**

(45) Publication of the grant of the patent:
**29.05.91 Bulletin 91/22**

(84) Designated Contracting States:
**AT BE CH DE ES FR GB GR IT LI LU NL SE**

(56) References cited:
**US-A- 4 294 575**

(73) Proprietor: **CIBA-GEIGY AG**
**Klybeckstrasse 141**
**CH-4002 Basel(CH)**

(72) Inventor: **Tsao, Fu-Pao**
**1361 Pinehurst Hunt**
**Lawrenceville, GA 30245(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to an improved method of disinfecting a soft contact lens with a stabilized aqueous hydrogen peroxide solution having a hydrogen peroxide content from 0.5 to 6 % by weight hydrogen peroxide wherein the stabilized solution contains from 0.003 to 0.03 % by weight of diethylene triamine penta(methylenephosphonic acid) of the formula

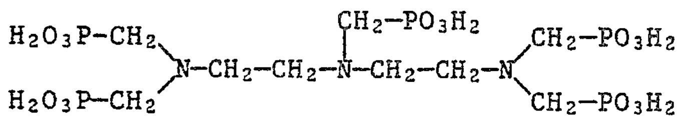

or a physiologically compatible salt thereof.

Soft contact lenses are characteristically prepared from hydrophilic polymers, such as polymers of hydroxyethyl methacrylate (HEMA), crosslinked with a conventional crosslinking agent, such as ethylene glycol dimethacrylate (EGDMA), or more complex copolymer systems including copolymers of HEMA, EGDMA, methacrylic acid and/or poly-N-vinylpyrrolidone, and the like. Other hydrophilic monomers conventionally employed in varying amounts in the manufacture of soft contact lenses include, for example, N-vinylpyrrolidone, glyceryl methacrylate, diethylene glycol monomethacrylate, triethylene glycol monomethacrylate, allyl 2-hydroxyethyl ether, acrylic acid, acrylamide, N,N-dimethylacrylamide, and the like. Other conventional crosslinking agents commonly employed include, inter alia, diallyl ether, divinyl benzene, diethylene glycol dimethacrylate, triethylene glycol dimethacrylate, diallyl succinate, allyl methacrylate, glycerin tri-methacrylate, and the like. Moreover, various amounts of relatively hydrophobic monomer units can be employed in the manufacture of soft contact lens materials, as long as the final copolymer network exhibits the desired hydrophilic characteristics. Typical hydrophobic monomers include methyl methacrylate, glycidyl methacrylate, N-(1,1-dimethyl-3-oxobutyl)acrylamide, siloxane methacrylates, perfluoroalkyl methacrylates, perfluoroalkoxyperfluoroalkyl methacrylates, and the like. In general, such lenses exhibit marked hydrophilic properties and, when wet, absorb water and are soft and flexible.

While these lenses are not actually perforate, they do have a sufficient degree of molecular porosity to permit water, oxygen and tear fluids to permeate the lens structure. In order for the disinfection of such lenses to be effective after they have been worn, it is important that contaminants be removed from both surfaces, and the interior of the lens, to the extent contaminants are present therein. Hydrogen peroxide in the form of a dilute solution, e.g. 0.5 to 6 % by weight in water, is known to be effective for use with contact lenses in order to kill any contaminating microorganisms.

One drawback with unstabilized dilute hydrogen peroxide solutions, however, is that without the use of a stabilizer or a combination of stabilizers, the aqueous peroxide solutions characteristically decompose over a period of time. The rate at which such dilute hydrogen peroxide solutions decompose will, of course, be dependent upon such factors as pH and the presence of trace amounts of various metal impurities, such as copper or chromium, which may act to catalytically decompose the same.

Moreover, at moderately elevated temperatures the rate of decomposition of such dilute aqueous hydrogen peroxide solutions is greatly accelerated.

A large variety of stabilizers has been proposed for use with hydrogen peroxide to deactivate trace catalytic impurities, including stannous salts, ethylene diamine tetraacetic acid, and the like.

For example, U.S. -A- 3,860,391 discloses bleaching compositions containing hydrogen peroxide and, as a stabilizer, amino lower alkylene polyphosphonates, including diethylene triamine penta-(methylenephosphonic acid) or salts thereof, and/or hydroxy alkane phosphates, with or without additional stabilizer constituents, and adjusted to a pH of between about 9.0 and 12.0 with, e.g. sodium hydroxide, for the bleaching of cellulose materials. Exemplified are compositions having a pH of 12.0.

Unfortunately, such highly basic compositions are undesirable in a contact lens environment and especially in the disinfection of contact lenses.

Thus, it is essential that the hydrogen peroxide containing solutions employed be, upon disinfection of the contact lenses and decomposition of the hydrogen peroxide, sufficiently compatible with the ocular environment so as not to occasion ocular irritation or damage if the disinfected contact lens containing the residual solution absorbed by the lens, is placed into the eye.

Caustic compositions, for example, can occasion severe ocular irritation and damage to the ocular tissue.

It is thus an object of the present invention to provide a method of disinfecting a soft contact lens with a

stabilized aqueous hydrogen peroxide solution having a pH of 5.5 to 7.5, employing diethylene triamine penta(methylenephosphonic acid) or a physiologically compatible salt thereof, as a stabilizer, such that upon disinfection of such contact lens and decomposition of residual hydrogen peroxide, the resulting solution, absorbed by and adhering to said lens, is physiologically tolerable to the ocular environment.

These and other objects of the present invention are apparent from the following more specific disclosures.

One embodiment of the present invention relates to a method of disinfecting a soft contact lens with a stabilized aqueous hydrogen peroxide solution having a pH between 5.5 and 7.5, a hydrogen peroxide content of between 0.5 and 6 % by weight, and containing between 0.003 and 0.03 % by weight of diethylene triamine penta(methylenephosphonic acid) or a physiologically compatible salt thereof, as a stabilizer, such that the solution, upon decomposition of the hydrogen peroxide, is physiologically tolerable to the ocular environment.

Physiologically compatible salts of diethylene triamine penta(methylenephosphonic acid) include, for example, water soluble salts with conventional pharmaceutically acceptable cationic moieties, including the alkali metal, alkaline earth metal, ammonium and amine cations. Suitable amine salts include, for example, mono-, di- and tri-lower alkyl amines, such as methylamine, ethylamine, diethylamine, triethylamine, dimethylamine, trimethylamine, propylamine, and the like; and mono-, di- and tri-lower hydroxyalkyl amines, such as ethanolamine, diethanolamine, triethanolamine, glucamine, 2-hydroxypropylamine, and the like.

By "lower", e.g. in the context of an alkyl group, is meant a group having up to 6 carbon atoms, preferably up to 4 carbon atoms.

Preferably, the concentration of hydrogen peroxide in the stabilized solution is between 2 % and 6 % by weight, based upon the weight of the formulation.

Preferably, diethylene triamine penta(methylenephosphonic acid) or a salt thereof is present in the stabilized composition in an amount between 0.006 and 0.02 % by weight of the composition, and most preferably between 0.006 and 0.0120 % by weight of the composition.

If desired, additional conventional stabilizers may be employed in conjunction with the diethylene triamine penta(methylenephosphonic acid) or salt stabilizer in accordance with the present invention.

Suitable conventional stabilizers include: water soluble stannates, such as alkali metal or ammonium stannates, for example sodium stannate, alone or in combination with a water soluble phosphate, polyphosphate or metaphosphate salt, such as an alkali metal or ammonium salt thereof; or an amino polycarboxylic acid chelating agent, such as ethylene diamine tetraacetic acid, nitrilo triacetic acid or a water soluble salt thereof, such as an alkali metal or ammonium salt, especially the sodium salt, or mixtures thereof. Where such additional stabilizers are employed, they are generally employed in a physiologically tolerable amount, e.g. in an amount of 0.002 to 0.1 % by weight.

However, the presence of additional stabilizers is on no account essential. In contrast to the prior art where diethylene triamine penta(methylenephosphonic acid), when used as a stabilizer of hydrogen peroxide solutions, as in U.S. -A- 4,294,575; 4,525,291 or 4,614,646, is generally used in a combination with other stabilizers, the presence of additional stabilizers is preferably limited in the methods according to the present invention.

Thus, a preferred embodiment of the invention is a method as defined hereinbefore wherein diethylene triamine penta(methylenephosphonic acid) or a physiologically compatible salt thereof is the substantially effective stabilizer. In a more preferred embodiment the additional presence of stabilizing alkali metal polyphosphates or polyalkylenepolycarboxylic acids is excluded. An even more preferred embodiment relates to methods as defined hereinbefore wherein diethylene triamine penta(methylenephosphonic acid) or a physiologically compatible salt thereof is the only effective stabilizer.

The pH of the stabilized solution is, as stated above, between 5.5 and 7.5. Preferably, the pH of the stabilized hydrogen peroxide solution is between about 6 and 7, most preferably between 6.2 and 6.8. The pH can be adjusted as desired by incorporation of suitable amounts of acid or base of a physiologically tolerable nature in the amounts employed.

Also, there may be present in the stabilized hydrogen peroxide solution according to the present invention, one or more conventional, substantially inert, physiologically acceptable tonicity enhancing agents. Suitably such agents include, for example alkali metal halides, phosphates, hydrogen phosphates, and borates. Preferred are sodium chloride, sodium phosphate monobasic and sodium phosphate dibasic. The function of such tonicity enhancing agents is to increase the comfort level of the solution, after decomposition of the hydrogen peroxide during or subsequent to contact lens disinfection, which adheres to the contact lens, in the eye of the patient.

Preferably sufficient tonicity enhancing agents are present in the solution, such that, upon decomposition of the hydrogen peroxide therein, the resulting solution is substantially isotonic, e.g. substantially

equivalent to a 0.9 % by weight aqueous sodium chloride solution.

In use, the contact lens to be disinfected is removed from the eye of the patient and placed into a solution of the stabilized hydrogen peroxide to disinfect the lens. Upon disinfection the hydrogen peroxide is decomposed, e.g. by the use of a conventional catalyst, such as a platinum catalyst on a support, or catalase, or by the use of a physiologically acceptable hydrogen peroxide decomposition agent, such as a physiologically acceptable reducing agent, including pyruvic acid or suitable salts thereof, e.g. the sodium salt. The lens can thereafter be stored in the resulting solution until reinsertion into the eye.

In general, the stabilized hydrogen peroxide solutions used in the method of the present invention are characterized by their extraordinary stability, even under accelerated conditions, for example by heating the solutions to 100 °C for 24 hours. Moreover, the instant compositions are characterized by their physiological tolerability subsequent to hydrogen peroxide decomposition.

The following examples are presented for illustrative purposes and are not intended to limit the scope of this invention. All parts are by weight unless otherwise indicated.

Example 1:

Into 80 ml distilled deionized water there is added 0.8655 g sodium chloride, 0.0622 g sodium phosphate dibasic (anhydrous), 0.0072 g sodium phosphate monobasic (monohydrate) and 0.006 g diethylene triamine penta(methylenephosphonic acid) with stirring.

To the resulting solution there is added 31 % aqueous hydrogen peroxide in an amount of 10 ml, and sufficient distilled deionized water is added to result in a total volume of 100 ml. Thereupon the pH is adjusted to 6.5 by the dropwise addition of dilute aqueous hydrogen chloride or sodium hydroxide.

The resulting stabilized 3 % hydrogen peroxide is then heated to 100 °C for a period of 24 hours. The solution possesses a "hot stability" of above 95 %, i.e. the amount of the hydrogen peroxide present in the after-heated sample related to the before-heated sample exceeds 95 %.

Example 2:

The procedure of example 1 is duplicated, except that typical deionized water is employed. Again, the hot stability of the resulting stabilized hydrogen peroxide solution is above 95 %.

Example 3:

The procedure of example 1 is duplicated, except that conventional soft tap water is employed. Once again, the hot stability of the resulting stabilized hydrogen peroxide solution is above 95 %.

## Claims

1. A method of disinfecting a soft contact lens with a stabilized aqueous hydrogen peroxide solution having a pH between 5.5 and 7.5, a hydrogen peroxide content of between 0.5 and 6 % by weight, and containing between 0.003 and 0.03 % by weight of diethylene triamine penta(methylenephosphonic acid) or a physiologically compatible salt thereof, as a stabilizer, such that the solution, upon decomposition of the hydrogen peroxide, is physiologically tolerable to the ocular environment.

2. A method according to claim 1, wherein said solution has a hydrogen peroxide content of 2 to 6 % by weight, based upon the weight of solution.

3. A method according to claim 1, wherein the pH is between 6 and 7.

4. A method according to claim 1, wherein the pH is between 6.2 and 6.8.

5. A method according to claim 1, in which the concentration of diethylene triamine penta-(methylenephosphonic acid) is between 0.006 and 0.02 % by weight of the composition.

6. A method according to claim 1, wherein said solution, upon decomposition of the hydrogen peroxide therein, is substantially isotonic.

7. A method according to claim 2, wherein said solution, upon decomposition of the hydrogen peroxide therein, is substantially isotonic.

8. A method according to claim 1, wherein, upon disinfection of said lens, the hydrogen peroxide in said solution is decomposed by the use of an effective amount of a hydrogen peroxide decomposition catalyst or agent.

9. A method according to claim 8, wherein upon hydrogen peroxide decomposition, said solution is substantially isotonic.

10. A method according to claim 1, in which said solution additionally contains between 0.002 and 0.1 % of a water soluble stannate or amino polycarboxylic acid chelating agent.

11. A method according to claim 1, wherein in said solution the presence of alkali metal polyphosphates or polyalkylene polycarboxylic acids as additional stabilizer is excluded.

12. A method according to claim 1, wherein in said solution diethylene triamine penta-(methylenephosphonic acid) or a physiologically compatible salt thereof is the only effective stabilizer.

**Revendications**

1. Procédé de désinfection d'une lentille de contact souple avec une solution aqueuse de peroxyde d'hydrogène stabilisée ayant un pH de 5,5 à 7,5, une teneur en peroxyde d'hydrogène comprise entre 0,5 et 6% en poids, et contenant entre 0,003 et 0,03% en poids de diéthylène triamine penta(acide méthylènephosphonique) ou d'un sel physiologiquement compatible de celui-ci, comme agent stabilisant, tel que la solution, lors de la décomposition du peroxyde d'hydrogène, est physiologiquement tolérable pour l'environnement oculaire.

2. Procédé selon la revendication 1, caractérisé en ce que ladite solution a une teneur en peroxyde d'hydrogène de 2 à 6% en poids, par rapport au poids de la solution.

3. Procédé selon la revendication 1, caractérisé en ce que le pH est entre 6 et 7.

4. Procédé selon la revendication 1, caractérisé en ce que le pH est entre 6,2 et 6,8.

5. Procédé selon la revendication 1, caractérisé en ce que la concentration de diéthylène triamine penta-(acide méthylènephosphonique) est comprise entre 0,006 et 0,02% en poids de la composition.

6. Procédé selon la revendication 1, caractérisé en ce que ladite solution, lors de la décomposition du peroxyde d'hydrogène qui s'y trouve, est pratiquement isotonique.

7. Procédé selon la revendication 2, caractérisé en ce que ladite solution, lors de la décomposition du peroxyde d'hydrogène qui s'y trouve, est pratiquement isotonique.

8. Procédé selon la revendicaiton 1, caractérisé en ce que lors de la désinfection de ladite lentille le peroxyde d'hydrogène dans ladite solution est décomposé par l'utilisation d'une quantité efficace de catalyseur ou d'agent de décomposition du peroxyde d'hydrogène.

9. Procédé selon la revendication 8, caractérisé en ce que ladite solution, lors de la décomposition du peroxyde d'hydrogène, est pratiquement isotonique.

10. Procédé selon la revendication 1, caractérisé en ce que ladite solution contient en outre entre 0,002 et 0,1% d'un stannate hydrosoluble ou d'un acide amino polycarboxylique hydrosoluble en tant qu'agent de chélation.

**11.** Procédé selon la revendication 1, caractérisé en ce que la présence de phosphates de métal alcalin ou d' acides polyalkylène polycarboxyliques en tant qu'agent stabilisant additionnel est exclue dans ladite solution.

**12.** Procédé selon la revendication 1, caractérisé en ce que le diéthylène triamine penta(acide méthylènephosphonique) ou un sel physiologiquement compatible de celui-ci est le seul agent stabilisant efficace présent dans ladite solution.

## Ansprüche

**1.** Verfahren zum Desinfizieren einer weichen Kontaktlinse mit einer stabilisierten wäßrigen Wasserstoffperoxidlösung mit einem pH zwischen 5,5 und 7,5, einem Wasserstoffperoxidgehalt zwischen 0,5 und 6 Gew.-%, und zwischen 0,003 und 0,03 Gew.-% Diethylentriaminpenta(methylenphosphonsäure) oder ein physiologisch kompatibles Salz davon als Stabilisator enthaltend, so daß die Lösung nach Zersetzung des Wasserstoffperoxids für das Augenmilieu physiologisch verträglich ist.

**2.** Verfahren nach Anspruch 1, worin diese Lösung einen Wasserstoffperoxidgehalt von 2 bis 6 Gew.-% bezogen auf das Gewicht der Lösung hat.

**3.** Verfahren nach Anspruch 1, worin der pH-Wert zwischen 6 und 7 liegt.

**4.** Verfahren nach Anspruch 1, worin der pH-Wert zwischen 6,2 und 6,8 liegt.

**5.** Verfahren nach Anspruch 1, in dem die Konzentration an Diethylentriaminpenta-(methylenphosphonsäure) zwischen 0,006 und 0,02 Gew.-% der Zusammensetzung beträgt.

**6.** Verfahren nach Anspruch 1, worin diese Lösung nach Zersetzung des Wasserstoffperoxids darin im wesentlichen isotonisch ist.

**7.** Verfahren nach Anspruch 2, worin diese Lösung nach Zersetzung des Wasserstoffperoxids darin im wesentlichen isotonisch ist.

**8.** Verfahren nach Anspruch 1, worin nach der Desinfizierung der Linsen das Wasserstoffperoxid in dieser Lösung durch Verwendung einer wirksamen Menge eines Wasserstoffperoxid-Zersetzungskatalysators oder -mittels zersetzt wird.

**9.** Verfahren nach Anspruch 8, worin nach der Wasserstoffperoxidzersetzung die Lösung im wesentlichen isotonisch ist.

**10.** Verfahren nach Anspruch 1, in dem besagte Lösung zusätzlich zwischen 0,002 und 0,1% eines wasserlöslichen Stannats oder Aminopolcarbonsäure-Chelatbildners enthält.

**11.** Verfahren nach Anspruch 1, worin in dieser Lösung die Gegenwart von Alkalimetallpolyphosphaten oder Polyalkylenpolycarbonsäuren als zusätzlicher Stabilisator ausgeschlossen ist.

**12.** Verfahren nach Anspruch 1, worin in der Lösung Diethylentriaminpenta(methylenphosphonsäure) oder ein physiologisch kompatibles Salz davon der einzige wirksame Stabilisator ist.